# EUROPÄISCHE PATENTSCHRIFT

(11) **EP 1 680 405 B1**
(45) Veröffentlichungstag und Bekanntmachung des Hinweises auf die Patenterteilung: **07.01.2009**
(21) Anmeldenummer: 04765884.4
(22) Anmeldetag: 08.10.2004
(51) Int. Cl.: C07D 223/10, C07D 223/12, A61K 31/55, A61P 35/00

(54) **BENGAMIDE-DERIVATE UND DEREN VERWENDUNG ZUR BEHANDLUNG VON KREBSERKRANKUNGEN**
BENGAMIDE DERIVATIVES AND USE THEREOF FOR THE TREATMENT OF CANCER
DÉRIVÉS DE BENGAMIDE ET LEUR UTILISATION POUR TRAITER LES MALADIES CANCEREUSES

(30) Priorität: 24.10.2003 DE 10349669
(43) Veröffentlichungstag der Anmeldung: 19.07.2006
(60) Teilanmeldung: 07007021.4
(73) Patentinhaber: Sanofi-Aventis Deutschland GmbH, 65929 Frankfurt am Main (DE)
(72) Erfinder: HOFFMANN, Holger, 64646 Heppenheim (DE); HAAG-RICHTER, Sabine, Patentabteilung, K 801, 65926 Frankfurt (DE); KURZ, Michael, 65926 Frankfurt am Main (DE); TIETGEN, Heiko, 55130 Mainz (DE)
(86) Internationale Anmeldenummer: PCT/EP2004/011244
(87) Internationale Veröffentlichungsnummer: WO 2005/044803

(56) Entgegenhaltungen:
- WO-A-01/85697
- JP-A- 2004 262 793
- J.ORG.CHEM., Bd. 66, 2001, Seiten 1733-1741, XP002310387
- J.MED.CHEM., Bd. 44, 2001, Seiten 3692-3699, XP002310388
- JOURNAL OF NATURAL PRODUCTS, Bd. 62, 1999, Seiten 1691-1693, XP002310389

## Beschreibung

Krebs ist eine zumeist tödlich verlaufende Erkrankung von Mensch und Tier, die durch das unkontrollierte Wachsen von körpereigenen Zellen verursacht wird. Krebs ist die Bezeichnung für die Bildung von bösartigen Geschwülsten (Malignome), von Neoplasmen (Tumoren bzw. Carcinoma) oder für die maligne Entartung sowie Reifungsstörung weißer Blutzellen (Leukämie, Blutkrebs). Krebs- oder Tumorzellen entstehen durch Umwandlung körpereigener Zellen. Die Bösartigkeit der Krebszelle drückt sich in der Autonomie des Wachstums aus, das heißt in ihrer Fähigkeit, ungehemmt und ohne Einordnung in den Bauplan der Organe und unter Gewebszerstörung infiltrierend zu wachsen. Ein sicheres Zeichen der Malignität ist die Bildung tumorferner Absiedlungen (Metastasen) nach hämatogener oder lymphogener Ausbreitung von Tumorzellen. Krebs gehört zu den häufigsten Todes-ursachen des Menschen und deshalb besteht ein großer Bedarf an Methoden und Mitteln zur Heilung oder Behandlung von malignen Entartungen.

Die Möglichkeit einer Therapie maligner Tumoren umfasst neben der - wenn möglich radikalen - operativen Entfernung des Tumors, die radiologische Therapie mit Röntgenstrahlen, α-, β-, γ-Strahlen, die Immuntherapie und die Chemotherapie. Die Immuntherapie ist derzeit nur in eingeschränktem Maß anwendbar. Unter der Chemotherapie von Tumoren versteht man die Gabe von Zellgiften (Cytostatika) zur Behandlung von Tumoren und von verbliebenen Tumorzellen zumeist nach lokaler chirurgischer Behandlung oder Bestrahlung. Diese Stoffe greifen spezifisch in bestimmte Vorgänge der Zellteilung ein, so dass Gewebe mit hohem Anteil an sich teilenden Zellen, wie das rasch wachsende Tumorgewebe, empfindlicher reagieren. Zum Einsatz kommen alkylierende Verbindungen, wie z. B. das Cyclophosphamid, Antimetabolite, wie das Methotrexat, Alkaloide, wie das Vincristin und Antibiotika, wie das Daunomycin oder das Adriamycin. All diese Agenzien haben aber aufgrund von massiven Nebenwirkungen große Nachteile, so dass der Tod der Erkrankten nur hinausgezögert, nicht jedoch abgewendet wird. Zudem treten bei entarteten (Krebs-) Zellen Resistenzen gegen die angewandten Mittel auf, die derzeitigen Medikamente wirken dann nicht mehr cytostatisch, wohl aber toxisch infolge der Nebenwirkungen. Zudem hat sich gezeigt, dass eine kombinierte bzw. sequentielle Anwendung von Cytostatika die Wirksamkeit eines einzelnen Cytostatikums (Monotherapie) übertrifft und dadurch möglich ist, dass sich die erheblichen Nebeneffekte bei der Polychemotherapie nicht addieren. Aus all diesen Gründen werden neue Chemotherapeutika dringend benötigt und deshalb weltweit gesucht.

Als erste Beispiele der Bengamide wurden die am Caprolactamring dodecanoylsubstituierten Bengamide A und B aus dem Meeresschwamm *Jaspis cf. Coriacea* (Familie *Coppatiidae*, Ordnung *Choristida B Astrophorida*) isoliert (Adamczewski et al., J. Org. Chem. 1986, 51, 4497-4498) und als biotoxisch gegen eukaryotische Zellen, Nematoden und Bakterien beschrieben.

Beispiele für Bengamide-Derivate, bei denen eine Antitumoraktivität nachgewiesen wurde, sind das Bengamide E 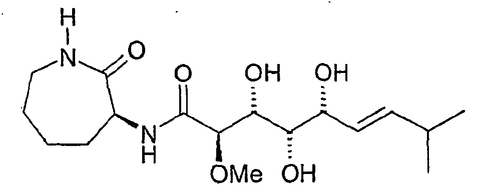 und dessen N-methyliertes Derivat Bengamide F. Bengamide E hemmt die Zellproliferation dadurch, dass die Zellteilung am G1/S Restriktionspunkt und in der G2/M Phase des Zell-Zyklus gestoppt wird. Bengamide B Derivate hemmen die Zellproliferation von MDA-MB-435 Brustkrebszellen (Kinder et al., J. Med. Chem. 2001, 44, 3692-3699).

Den bekannten Bengamide-Derivaten ist gemeinsam, dass sie aus Meeresschwämmen der Gattung Jaspis sp. oder Pachastrissa sp. isoliert wurden (Thale et al., J. Org. Chem. 2001, 66, 1733-1741).

Es wurde nun gefunden, dass der Mikroorganismus-Stamm *Myxococcus virescens* ST200611 (DSM 15898), neuartige Bengamide-Derivate zu bilden vermag, die die Zellproliferation in geringen Konzentrationen hemmen und demzufolge für die Behandlung und/oder Prophylaxe von Krebserkrankungen geeignet sind. Die vorliegende Erfindung betrifft eine Verbindung der Formel (I), 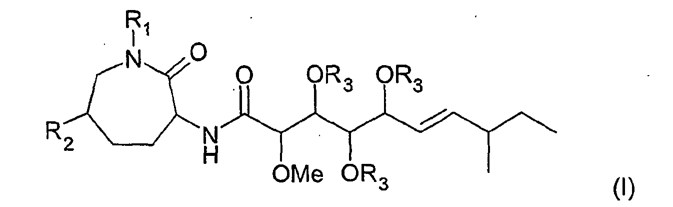 wobei
R₁ H oder (C₁-C₆)-Alkyl,
R₂ H oder OH, und
R₃ H oder -C(=O)-(C₁-C₆)-Alkyl bedeutet,
oder ein physiologisch verträgliches Salz einer Verbindung der Formel (I).

Unabhängig voneinander ist R₁ vorzugsweise H oder Methyl, und R₃ vorzugsweise H.

Bevorzugt betrifft die Erfindung eine Verbindung der Formel (I), worin
R₁ H oder Methyl,
R₂ H oder OH, und
R₃ H bedeutet.
(C₁-C₆)-Alkyl bedeutet eine geradkettige oder verzweigte Alkylgruppe mit 6 Kohlenstoffatomen, beispielsweise Methyl (Me), Ethyl, n-Propyl, iso-Propyl, tert-Butyl oder n-Hexyl, vorzugsweise Methyl.

Ferner betrifft die Erfindung eine Verbindung der Formel (I) gekennzeichnet durch eine Verbindung der Formel (II), 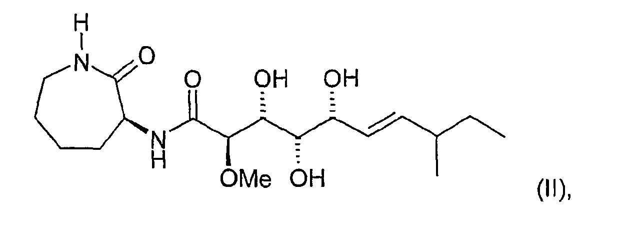 eine Verbindung der Formel (III) 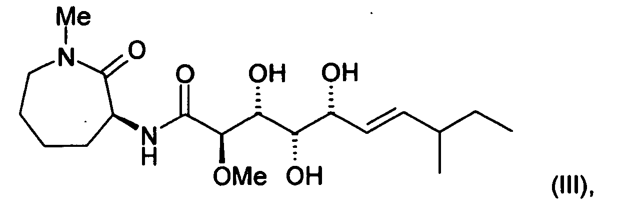 und eine Verbindung der Formel (IV)

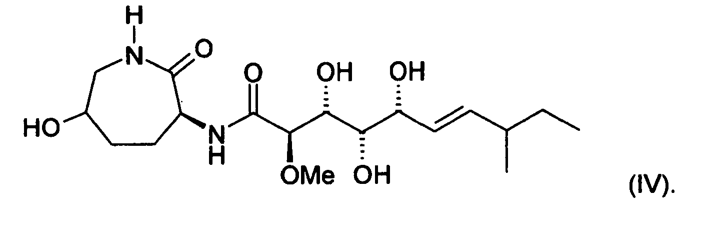

Die vorliegende Erfindung betrifft weiterhin physiologisch verträgliche Salze der Verbindungen der Formel (I)

Chiralitätszentren in den Verbindungen der Formel (I) können, wenn nicht anders angegeben, in der R- oder in der S-Konfiguration vorliegen. Die Erfindung betrifft sowohl die optisch reinen Verbindungen als auch Stereoisomerengemische wie Enantiomerengemische und Diastereomerengemische.

Unter physiologisch verträglichen Salzen von Verbindungen der Formel (I) versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Verbindungen der Formel (I) können hergestellt werden, indem
1. der Stamm *Myxococcus virescens* ST200611 (DSM 15898) oder einer seiner Varianten und/oder Mutanten unter geeigneten Bedingungen in einem Kulturmedium fermentiert wird, bis sich eine oder mehrere der Verbindungen der Formel (I) in dem Kulturmedium anhäuft,
2. eine Verbindung der Formel (I) aus dem Kulturmedium isoliert wird, und
3. die Verbindung der Formel (I) gegebenenfalls derivatisiert und/oder gegebenenfalls in ein physiologisch verträgliches Salz umgewandelt wird.

Das Kulturmedium ist eine Nährlösung oder ein Festmedium mit mindestens einer üblichen Kohlenstoff- und Stickstoffquelle sowie den üblichen anorganischen Salzen. Wird dem Kulturmedium Hydroxylysin zugesetzt, produziert der Stamm *Myxococcus virescens* ST200611 (DSM 15898) durch Verdau von Hydroxylysin eine Verbindung der Formel (I), in der R₂ gleich OH ist.

Das Verfahren kann für die Fermentation im Labormaßstab (Milliliter- bis Litermaßstab) und für den industriellen Maßstab (Kubikmetermaßstab) eingesetzt werde.

Als Kohlenstoffquellen für die Fermentation eignen sich assimilierbare Kohlenhydrate und Zuckeralkohole, wie Glukose, Laktose, Saccharose oder D-Mannit sowie kohlenhydrathaltige Naturprodukte, wie z.B. Malzextrakt oder Hefextrakt. Stickstoffhaltige Nährstoffe sind beispielsweise Aminosäuren; Peptide und Proteine sowie deren Abbauprodukte, beispielsweise Casein, Peptone oder Tryptone; Fleischextrakte; Hefeextrakte; Gluten; gemahlene Samen, beispielsweise von Mais, Weizen, Bohnen, Soja oder der Baumwollpflanze; Destillationsrückstände der Alkoholherstellung; Fleischmehle; Hefeextrakte; Ammoniumsalze; Nitrate. Vorzugsweise ist die Stickstoffquelle ein oder mehrere synthetisch bzw. biosynthetisch gewonnene Peptide. Anorganische Salze sind beispielsweise Chloride, Carbonate, Sulfate oder Phosphate der Alkali- oder Erdalkalimetalle, Eisen, Zink, Kobalt und Mangan enthalten. Spurenelemente sind beispielsweise Kobalt und Mangan.

Geeignete Bedingungen für die Bildung der erfindungsgemäßen Bengamide sind wie folgt: Die Bildung der erfindungsgemäßen Bengamide verläuft bevorzugt in einem Kulturmedium, die 0.05 bis 5 %, bevorzugt 0.1 bis 2.5% Hefextrakt; 0.2 bis 5.0%, bevorzugt 0,1 bis 2 % Casitone; 0.02 bis 1.0%, bevorzugt 0.05 bis 0.5 % CaCl₂ x 2 H₂O; 0.02 bis 1.5%, bevorzugt 0.05 bis 0.7% MgSO₄ x 7H₂O sowie 0.00001% bis 0.001 % Cyanocobalamin enthält. Die Angaben in Prozent sind jeweils bezogen auf das Gewicht der gesamten Nährlösung.

Die Kultivierung des Mikroorganismus erfolgt aerob, also beispielsweise submers unter Schütteln oder Rühren in Schüttelkolben oder Fermentern oder auf Festmedium, gegebenenfalls unter Einführen von Luft oder Sauerstoff. Sie kann in einem Temperaturbereich von etwa 18 bis 35°C, vorzugsweise bei etwa 20 bis 32°C, insbesondere bei 27 bis 30°C durchgeführt werden. Der pH-Bereich sollte zwischen 4 und 10 liegen, vorzugsweise zwischen 6.5 und 7.5. Man kultiviert den Mikroorganismus unter diesen Bedingungen im allgemeinen über einen Zeitraum von 2 bis 10 Tagen, vorzugsweise 72 bis 168 Stunden. Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10 bis 1:100, überimpft werden. Die Vorkultur erhält man z. B., indem man den Stamm in Form von vegetativen Zellen oder Fruchtkörpern in eine Nährlösung überimpft und etwa 20 bis 120 Stunden, bevorzugt 48 bis 96 Stunden, wachsen lässt. Vegetative Zellen und/oder Fruchtkörper können beispielsweise erhalten werden, indem man den Stamm etwa 1 bis 15 Tage, bevorzugt 4 bis 10 Tage, auf einem festen oder flüssigen Nährboden, beispielsweise Hefeagar wachsen lässt.

Die Isolierung bzw. Aufreinigung eines Bengamide-Derivates der Formel (I) aus dem Kulturmedium erfolgt nach bekannten Methoden unter Berücksichtigung der chemischen, physikalischen und biologischen Eigenschaften der Naturstoffe. Zum Testen der Konzentration der jeweiligen Bengamide-Derivate im Kulturmedium oder in den einzelnen Isolierungsstufen wurde HPLC verwendet, wobei die Menge der gebildeten Substanz zweckmäßig mit einer Eichlösung verglichen wurde.

Zur Isolierung werden die Kulturbrühe oder die Kultur samt Festmedium lyophilisiert, anschließend werden die Bengamide-Derivate vom Lyophilisat mit einem organischen Lösungsmittel, beispielsweise Methanol oder 2-Propanol extrahiert. Die organische Lösungsmittelphase enthält die erfindungsgemäßen Naturstoffe, sie wird gegebenenfalls im Vakuum konzentriert und weiter aufgereinigt.

Die weitere Aufreinigung einer oder mehrerer erfindungsgemäßer Verbindungen erfolgt durch Chromatographie an geeigneten Materialien, vorzugsweise z. B. an Molekularsieben, an Kieselgel, Aluminiumoxid, an lonenaustauschern oder an Adsorberharzen bzw. an Umkehrphasen (reversed phase, RP). Mit Hilfe dieser Chromatographie werden die Bengamide-Derivate getrennt. Die Chromatographie der Bengamide-Derivate erfolgt mit gepufferten wässrigen Lösungen oder Gemischen von wässrigen und organischen Lösungen.

Unter Gemischen von wässrigen oder organischen Lösungen versteht man alle mit Wasser mischbaren organischen Lösemittel, vorzugsweise Methanol, 2-Propanol und Acetonitril, in einer Konzentration von 5 bis 95 % Lösemittel, vorzugsweise 5 bis 40 % Lösemittel oder auch alle gepufferten wässrigen Lösungen, die mit organischen Lösemitteln mischbar sind. Die zu verwendenden Puffer sind die gleichen wie oben angegeben.

Die Trennung der Bengamide-Derivate aufgrund ihrer unterschiedlichen Polarität erfolgt mit Hilfe der Reversed Phase Chromatographie, beispielsweise an MCI® (Adsorberharz von Mitsubishi, Japan) oder Amberlite XAD® (TOSOHAAS), oder an weiteren hydrophoben Materialien, wie zum Beispiel an RP-8- oder RP-18-Phasen. Außerdem kann die Trennung mit Hilfe der Normalphasen-Chromatographie, zum Beispiel an Kieselgel, Aluminiumoxid und ähnlichen erfolgen.

Die Chromatographie der Bengamide-Derivate erfolgt mit gepufferten, basischen oder angesäuerten wässrigen Lösungen oder Gemischen von wässrigen Lösungen mit Alkoholen oder anderen, mit Wasser mischbaren organischen Lösemitteln. Als organisches Lösemittel wird vorzugsweise Acetonitril und Methanol verwendet.

Unter gepufferten, basischen oder angesäuerten wässrigen Lösungen versteht man z.B. Wasser, Phosphatpuffer, Ammoniumacetat, Citratpuffer in einer Konzentration von bis zu 0,5 M sowie Ameisensäure, Essigsäure, Trifluoressigsäure, Ammoniak, Triethylamin oder allen handelsüblichen, dem Fachmann bekannten Säuren und Basen, vorzugsweise in einer Konzentration von bis zu 1 %. Bei gepufferten wässrigen Lösungen wird 0,1 % Ammoniumacetat besonders bevorzugt.

Chromatographiert wurde mit einem Gradienten, der mit 100 % Wasser begann und mit 100 % Lösemittel endete, vorzugsweise wurde ein linearer Gradient von 5 bis 95 % Acetonitril gefahren.

Alternativ kann auch eine Gelchromatographie oder die Chromatographie an hydrophoben Phasen erfolgen. Die Gelchromatographie wird an Polyacrylamid- oder Mischpolymergelen durchgeführt, wie z.B. Biogel-P 2® (Biorad) oder Fractogel TSK HW 40® (Merck, Deutschland). Die Reihenfolge der vorgenannten Chromatographien ist umkehrbar.

Sofern Bengamide als Diastereomere vorliegen, können diese über bekannte Methoden getrennt werden, beispielsweise durch Trennung über eine chirale Säule.

Die Derivatisierung der OH-Gruppen in der Seitenkette der Verbindungen der Formel (I) (R₃ jeweils gleich H) zu einer Acylgruppe (R₃ jeweils gleich -C(=O)-(C₁-C₆)-Alkyl) erfolgt nach ansich bekannten Methoden (J. March, Advanced Organic Chemistry, John Wiley & Sons, 4th Edition, 1992), beispielsweise durch Umsetzung mit einem Säureanhydrid. Die Umsetzung mit Acetanhydrid zu einer Verbindung der Formel (I), in der R₃ -C(=O)-CH₃ bedeutet, beschreibt beispielsweise Adamczeski et al., J. Am. Chem. Soc. 1989, 111, 647-654.

Die Alkylierung der NH-Gruppe im Caprolactamring einer Verbindung der Formel (I) (R₁ jeweils gleich H) erfolgt ebenfalls nach ansich bekannten Methoden (J. March, Advanced Organic Chemistry, John Wiley & Sons, 4th Edition, 1992), beispielsweise durch Reaktion mit Me₂CO₃ oder Me₂SO₄ zur Herstellung der entsprechenden N-methylierten Derivate, oder durch Reaktion mit (C₁-C₆)-Alkylbromid in Gegenwart einer Base.

Ein Isolat des Mikroorganismenstammes *Myxococcus virescens* ST200611 wurde bei der Deutschen Sammlung von Mikroorganismen und Zellkulturen GmbH (DSMZ), Mascheroder Weg 1 B, 38124 Braunschweig, Deutschland, nach den Regeln des Budapester Vertrages am 11.09.2003 unter der folgenden Nummer hinterlegt: DSM 15898.

Die vegetativen Zellen des Stammes DSM 15898 haben die für *Myxococcus virescens* charakteristische Stäbchenform. Auf festen Nährböden bildet *Myxococcus virescens* ST200611 (DSM 15898) orange-gelbe Fruchtkörper, welche runde Myxosporen enthalten.

Anstelle des Stammes *Myxococcus virescens* ST200611 (DSM 15898) können auch dessen Mutanten und/oder Varianten eingesetzt werden, die ein oder mehrere der erfindungsgemäßen Verbindungen synthetisieren.

Eine Mutante ist ein Mikroorganismus, in dem ein oder mehrere Gene des Genoms modifiziert wurden, wobei das Gen oder die Gene funktionell und vererbbar erhalten bleiben, die für die Fähigkeit des Organismus verantwortlich sind, die erfinderische Verbindung zu produzieren.

Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS); 2-Hydroxy-4-methoxy-benzophenon (MOB) oder N-Methyl-N'-nitro-N-nitrosoguanidin (MNNG) erzeugt werden, oder wie von Brock et al. in "Biology of Microorganism", Prentice Hall, Seite 238-247 (1984) beschrieben.

Eine Variante ist ein Phenotyp des Mikroorganismus. Mikroorganismen haben die Fähigkeit, sich an ihre Umgebung anzupassen und zeigen daher ausgeprägte physiologische Flexibilität. Bei der phenotypischen Anpassung sind alle Zellen des Mikroorganismus involviert, wobei die Art der Veränderung nicht genetisch konditioniert ist und unter veränderten Bedingungen reversibel ist (H. Stolp, Microbial ecology: organismus, habitats, activities. Cambridge University Press, Cambridge, GB, Seite 180, 1988).

Das Screening nach Mutanten und/oder Varianten, die ein oder mehrere der erfindungsgemäßen Verbindungen synthetisieren, erfolgt nach folgendem Schema:
- Lyophilisierung des Fermentationsmediums;
- Extraktion des Lyophilisats mit einem organischen Lösungsmittel
- Extraktion der Verbindung aus dem Kulturfiltrat mit Festphasen
- Analytik mittels HPLC, DC oder durch Testen der biologischen Wirksamkeit.

Die beschriebenen Fermentationsbedingungen gelten für *Myxococcus virescens* ST200611 (DSM 15898) sowie für Mutanten und/oder Varianten davon.

Zum Nachweis der Hemmung der Zellproliferation bedient man sich eines Testes, der auf der Bestimmung der intrazellulären ATP-Konzentration beruht. Verwendet werden können bekannte Tumor-Zelllinien wie beispielsweise Hep-G2 und Colo205. In diesem Test dient der ATP-Gehalt metabolisch aktiver Zellen dabei in einer Luciferasereaktion als Maß für die Zahl lebender Zellen.

Die Verbindungen der Formel (II)-(IV) wurden in einer Einzeldosis von 0.3 - 40 µM im Test eingesetzt und eine Dosisabhängigkeit als TC50-Wert angegeben, wobei (IIA) und (IIB) jeweils eine Diastereomer der Verbindung der Formel (II) bedeuten:

| Tabelle 1: Wirksamkeit der Bengamide im Zellproliferationstest als TC50-Wert in µM | | |
|---|---|---|
| Verbindung | Hep-G2 | Colo 205 |
| (II) | 16 | 27 |
| (IIA) | 17 | 33 |
| (IIB) | 6 | 10 |
| (III) | 9 | 15 |
| (IV) | >40 | 42 |
| Bengamide E (Vergleichsversuch) | 36 | 46 |
| Bengamide F (Vergleichsversuch) | 27 | 33 |

Gegenstand der Erfindung ist daher ferner die Verwendung der Verbindung der Formel (I) oder eines physiologisch verträglichen Salzes derselben zur Herstellung eines Arzneimittel in der Human- oder Tiermedizin, insbesondere zur Behandlung und/oder Prophylaxe von Krebserkrankungen. Vorzugsweise betrifft die Erfindung die Verwendung einer Verbindung der Formel (I) oder eines physiologisch verträglichen Salzes zur Herstellung eines Arzneimittel zur Behandlung von Brustkrebs, Darmkrebs, Magenkrebs, Leberkrebs, Gehirntumoren, Ovarialtumoren, Speiseröhrenkrebs, Nierenkrebs, Muskelzellkarzinom, insbesondere Kopf- und Nackenmuskelkarzinom.

Des weiteren betrifft die vorliegende Erfindung ein Arzneimittel mit einem Gehalt an mindestens einer Verbindung der Formel (I) oder eines physiologisch verträglichen Salzes davon, wobei die Verbindung oder die Verbindungen der Formel (I) als solche in Substanz verabreicht werden kann oder bevorzugt in Mischung mit einem oder mehreren der üblichen pharmakologisch geeigneten Träger- oder Hilfsstoffen vorliegen.

Die erfindungsgemäßen Verbindungen sind im festen Zustand und in Lösungen im pH-Bereich zwischen 2 und 9, insbesondere 5 und 7, stabil und lassen sich damit in übliche galenische Zubereitungen einarbeiten.

Die erfindungsgemäßen Arzneimittel können oral oder parenteral verabreicht werden, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise pharmakologisch geeignete Träger- oder Hilfsstoffe wie Spreng-, Binde-, Überzugs-, Quellungs-, Gleit-oder Schmiermittel, Geschmacksstoffe, Süßungsmittel oder Lösungsvermittler Verwendung finden, beispielsweise Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der Bengamide-Derivate der Formel (I) enthält. Bei festen Dosierungseinheiten wie Tabletten, Kapseln und Suppositorien kann diese Dosis bis zu etwa 500 mg, bevorzugt jedoch etwa 0.1 bis 200 mg, und bei Injektionslösungen in Ampullenform bis zu etwa 200 mg, vorzugsweise aber etwa 0.5 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die erfindungsgemäßen Arzneimittel werden dadurch hergestellt, dass man ein oder mehrere der erfindungsgemäßen Verbindungen der Formel (I) optional mit einem oder mehreren der üblichen Träger- oder Hilfsstoffe sowie in eine geeignete Darreichungsform bringt.

Die folgenden Beispiele sollen der näheren Erläuterung der Erfindung dienen, ohne die Breite der Erfindung in irgendeiner Weise zu begrenzen.

Sofern keine anderen Angaben gemacht werden, beziehen sich Prozentangaben auf das Gewicht, und Mischungsverhältnisse bei Flüssigkeiten auf das Volumen.

### Beispiel 1: Einlagerung bei -135°C von Myxococcus virescens ST200611 (DSM 15898)

Eine Agarplatte (1 % frische Bäckerhefe, 1 % CaCl₂ x 2H₂O, 20 mM HEPES, 0.00005% Cyanocobalamin, 1.5% Agar, pH 7.2) wird mit dem Stamm *Myxococcus virescens* ST200611 (DSM 15898), beimpft und ca. 7 Tage bei 30° C inkubiert. Die Zellen dieser Oberflächenkultur werden mit einem sterilen Spatel von der Agaroberfläche gekratzt, in 0.5 ml Casitonemedium (1% Casitone (Difco), 0.15% MgSO₄ x 7H₂O, pH 7.0) resuspendiert und bei -135°C gelagert.

### Beispiel 2: Herstellung einer Vorkultur im Erlenmeyerkolben von Myxococcus virescens ST200611 (DSM 15898)

100 ml Nährlösung (1 % frische Bäckerhefe, 1 % CaCl₂ x 2H₂O, 20 mM HEPES, 0.00005% Cyanocobalamin, pH 7.2) in einem sterilen 300 ml Erlenmeyerkolben werden mit dem Stamm *Myxococcus virescens* ST200611 (DSM 15898), beimpft und 4 Tage bei 30°C und 180 UpM auf einer rotierenden Schüttelmaschine inkubiert. 5 ml dieser Vorkultur werden anschließend für die Herstellung der Hauptkulturen verwendet.

### Beispiel 3: Herstellung einer flüssigen Hauptkultur Myxococcus virescens ST200611 (DSM 15898)

Ein steriler 300 ml Erlenmeyerkolben mit 100 ml der folgenden Nährlösung (0.5% Hefeextrakt, 0.5% Casitone, 0.1 % CaCl₂ x 2H₂O, 0.2% MgSO₄ x 7H₂O, 0.00005% Cyanocobalamin, pH 7.4) wird mit 5 ml einer Vorkultur (s. Beispiel 2) oder einer auf einer frischen Agarplatte gewachsenen Kultur (1 % frische Bäckerhefe, 1 % CaCl₂ x 2H₂O, 20mM HEPES, 0.00005% Cyanocobalamin, pH 7.2, zusätzlich 1.5% Agar) beimpft und auf einer Schüttelmaschine bei 180 UpM und 30° C inkubiert. Die maximale Produktion der erfindungsgemäßen Bengamide ist nach 72-96 Stunden erreicht. Zum Animpfen von 10 bis 200 L Fermentern genügt eine 72-96 Stunden alte Submerskultur (Animpfmenge ca. 5-10%) aus der gleichen Nährlösung wie in Beispiel 2 beschrieben.

### Beispiel 4: Herstellung einer flüssigen Hauptkultur Myxococcus virescens ST200611 (DSM 15898) unter Verfütterung von Hydroxylysin zur Produktion des Bengamid-Derivates (IV)

Ein steriler 300 ml Erlenmeyerkolben mit 100 ml der folgenden Nährlösung (0.5% Hefeextrakt, 0.5% Casitone, 0.1% CaCl₂ x 2H₂O, 0.2% MgSO₄ x 7H₂O, 0.00005% Cyanocobalamin, sowie 1 mM Hydroxylysin pH 7.4) wird mit 5 ml einer Vorkultur aus Beispiel 2 oder einer auf einer frischen Agarplatte gewachsenen Kultur (1 % frische Bäckerhefe, 1 % CaCl₂ x 2H₂O, 20 mM HEPES, 0.00005% Cyanocobalamin, pH 7.2, zusätzlich 1.5% Agar) beimpft und auf einer Schüttelmaschine bei 180 UpM und 30°C inkubiert. Die maximale Produktion des Bengamide Derivates (IV) ist nach 72-96 Stunden erreicht. Die Analytik erfolgte mittel HPLC-MS. Zum Animpfen von 10 bis 200 L Fermentern genügt eine 72-96 Stunden alte Submerskultur (Animpfmenge ca. 5-10%) aus der gleichen Nährlösung wie in Beispiel 2 beschrieben.

### Beispiel 5: Herstellung von Bengamid-Derivaten im Fermenter

Die 10 L und 30 L Fermenter wurden unter folgenden Bedingungen betrieben:

| Inokulum: | ca. 5% | ca. 9% |
|---|---|---|
| Fermenter: | 30 L | 10 L |
| Nährmedium: | siehe Beispiel 2 | siehe Beispiel 2 |
| Inkubationstemperatur: | 30°C | 30°C |
| Rührergeschwindigkeit: | 112 UpM | 150 UpM |
| Belüftung: | 8 L/Min | 4 L/min |
| pH Regulierung: | ab pH 7.8 auf pH 7.5 | ab pH 8.1 auf pH 7.5 |
| pO₂ Regulierung: | keine | keine |

Die pH Regulierung wurde immer mit 10% KOH, bzw. 10% H₂SO₄ durchgeführt. Durch wiederholte Zugabe von Clerol FBA 265 (Cognis Deutschland GmbH) kann die Schaumbildung unterdrückt werden. Das Produktionsmaximum wird nach ca. 72 bis 96 Stunden erreicht.

### Beispiel 6: Isolierung der Bengamide-Derivate (II) und (III), sowie Isolierung der Bengamide E und F (Vergleichsversuch) aus den Schüttelkulturen von Myxococcus virescens ST200611 (DSM 15898)

Nach Beendigung der Fermentation *Myxococcus virescens* ST200611 (DSM 15898) wurde die Kulturbrühe aus Beispiel 3 (30 L Kulturbrühe) mitsamt der Biomasse lyophilisiert und das Lyophilisat mit Methanol (2 x 5 L) extrahiert. Der Methanolextrakt wurde am Vakuum auf 1.2 L reduziert und anschließend auf eine vorbereitete Säule aufgetragen, die mit ca. 1.5 Liter CHP-20P Material (MCI® Gel, 75-150µ, Mitsubishi Chemical Corporation) gefüllt war. Eluiert wurde mit 95% Methanol. Der Säulendurchfluss (120 ml/min) wurde aufgefangen und im Vakuum auf ein Volumen 1.5 L reduziert.

### Beispiel 7: Vortrennung der Bengamide-Derivate (II) und (III), sowie Vortrennung der Bengamide E und F (Vergleichsversuch) durch RP-18 Chromatographie

1.5 L der nach Beispiel 6 gewonnenen Lösung wurde auf eine Phenomenex Luna® 10µ C18 (2) Säule (Größe: 50 mm x 250 mm) mit Vorsäule Luna® 10µ C18 (2) (Dimension: 21.2 mm x 60 mm) aufgetragen und mit einem Gradienten von 5% bis 95% Acetonitril in Wasser über 60 min eluiert (0.1 % Ammoniumacetat, pH 4.6 eingestellt mit Essigsäure). Der Durchfluss betrug 150 ml/min, die Fraktionsgröße 200 ml. Bengamide befanden sich in den Fraktionen 5-9, 10-11 und 12-14.

### Beispiel 8: Reinigung der Bengamide-Derivate (II) und (III), sowie Reinigung der Bengamide E und F (Vergleichsversuch)

Die einzelnen Fraktionen aus Beispiel 7 wurden lyophilisiert und nochmals mittels HPLC an einer Phenomenex Luna® 10 µm C18 (2) Säule (Dimension: 21 mm x 250 mm) mit Vorsäule XTerra® Prep MS C18 10 µm (Waters, Dimension: 19 x 10 mm) aufgereinigt. Eluiert wurde mit einem Gradienten von 5% nach 40% Acetonitril in Wasser über 40 min (mit Zusatz von 0. 1 % Ammoniumacetat, pH 8.8 eingestellt mit Triethylamin). Der Säulendurchfluss (50 ml/min) wurde fraktioniert aufgefangen (je 7,5 ml Fraktionen). Fraktion 5-9 aus Beispiel 7 enthielt die Verbindung der Formel (III) und ergab nach Chromatographie und Lyophilisieren 86 mg Bengamid E (Reinheit > 95%). Die Fraktion 10-11 aus Beispiel 7 ergab nach Chromatographie 145 mg Bengamid (II) (Reinheit > 95%, Diastereomeren-Gemisch 70/30) und 5 mg Bengamid F (Reinheit > 95%). Aus der Fraktion 12-14 aus Beispiel 7 wurden 35 mg Bengamid (III) (Reinheit> 95%) als Diastereomeren-Gemisch im Verhältnis 75:25 erhalten. Bei den Diastereomeren handelt es sich jeweils um die entsprechenden C-16 Epimere.

### Beispiel 9: Isolierung des Hydroxy-Bengamides (IV) aus den Schüttelkulturen von Myxococcus virescens ST200611 (DSM 15898)

Nach Beendigung der Fermentation *Myxococcus virescens* ST200611 (DSM 15898) wurde die Kulturbrühe aus Beispiel 5 (10 L Kulturbrühe) mitsamt der Biomasse lyophilisiert und das Lyophilisat mit Methanol (2 x 3 L) extrahiert. Der Methanolextrakt wurde am Vakuum auf 400 ml reduziert und anschließend auf eine vorbereitete Säule aufgetragen, die mit 0.6 Liter CHP-20P (MCl® Gel, 75-150µ, Mitsubishi Chemical Corporation) Material gefüllt war. Eluiert wurde mit 5% bis 95% Methanol in Wasser. Der Säulendurchfluss (100 ml/min) wurde über 60 min fraktioniert aufgefangen (0.5 min pro Fraktion). Die das gewünschte Derivat enthaltenden Fraktionen (Fraktion 45-109) wurden zusammengefasst und im Vakuum auf ein Volumen von 700 ml reduziert.

### Beispiel 10: Vorreinigung des Hydroxy-Bengamides (IV) durch RP-18 Chromatographie

Die Lösung aus Beispiel 9 wurde anschließend auf eine Phenomenex Luna® 10µ C18 (2) Säule (Größe: 50 mm x 250 mm) mit Vorsäule Phenomenex Luna® 10µ C18 (2) (Dimension: 21.2 mm x 60 mm) aufgetragen und mit einem Gradienten von 5% bis 40% Acetonitril in Wasser über 60 min eluiert (0.1% Ammoniumacetat, pH 8.8 eingestellt mit Triethylamin). Der Durchfluss betrug 150 ml/min, die Fraktionsgröße 225 ml. Fraktion 22 enthielt das gewünschte Bengamide-Derivat.

### Beispiel 11: Reinigung des Hydroxy-Bengamides (IV)

Fraktion 22 aus Beispiel 10 wurde lyopilisiert und nochmals mittels HPLC an einer Phenomenex Luna® 10 µm C18 (2) Säule (Dimension: 21 mm x 250 mm) mit Vorsäule Waters XTerra® Prep MS C18 10 µm (Dimension: 19 x 10 mm) aufgereinigt. Eluiert wurde mit einem Gradienten von 5 % nach 95% Acetonitril in Wasser über 60 min (mit Zusatz von 0.1% Ammoniumacetat, pH 4.6 eingestellt mit Essigsäure). Der Säulendurchfluss (50 ml/min) wurde fraktioniert aufgefangen (je 7,5 ml Fraktionen). Die Bengamide-haltigen Fraktionen (Fraktion 26-28) wurden vereinigt, entsalzt und gefriergetrocknet. Dabei wurden 7 mg Bengamide (IV) als Diastereomeren-Gemisch im Verhältnis 75:25 erhalten.

### Beispiel 12: Charakterisierung der Verbindung der Formel (II)

Summenformel: C₁₈H₃₂N₂O₆
Molekulargewicht: 372,47
Diastereomeren-Gemisch: 75:25

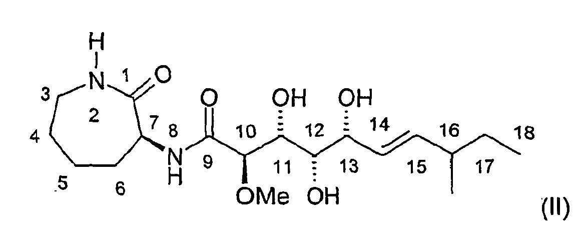

| Tabelle 2: NMR-chemische Verschiebungen von Bengamide (II), Diastereomerengemisch, c = 3 mg/ml in DMSO, 300 K. | | |
|---|---|---|
| Position | ¹H | ¹³C |
| 1 | - | 173.99 |
| 2 | 7.91 | - |
| 3 | 3.19/3.06 | 40.56 |
| 4 | 1.74/1.20 | 28.75 |
| 5 | 1.87/1.64 | 27.55 |
| 6 | 1.87/1.36 | 30.72 |
| 7 | 4.39 | 51.27 |
| 8 | 7.78 | - |
| 9 | - | 169.61 |
| 10 | 3.69 | 81.60 |
| 10-OMe | 3.25 | 57.32 |
| 11 | 3.58 | 70.72 |
| 11-OH | 4.46 | - . |
| 12 | 3.33 | 72.80 |
| 12-OH | 4.36 | - |
| 13 | 3.97 | 72.46 |
| 13-OH | 4.56 | - |
| 14 | 5.37 | 129.05 |
| 15 | 5.48 | 136.57 |
| 16 | 1.99 | 37.41 |
| 16-Me | 0.93 | 19.90 |
| 17 | 1.26 | 29.15 |
| 18 | 0.81 | 11.58 |

### Beispiel 13: Charakterisierung der Verbindung der Formel (III)

Summenformel: C₁₉H₃₄N₂O₆
Molekulargewicht: 386,49
Diastereomeren-Gemisch: 75/25

| Tabelle 3: NMR-chemische Verschiebungen von Bengamide (III), Diastereomerengemisch, c = 3 mg/ml in DMSO, 300 K. | | |
|---|---|---|
| Position | ¹H | ¹³C |
| 1 | - | 171.95 |
| 2 | 2.91 | 35.28 |
| 3 | 3.61/3.21 | 49.20 |
| 4 | 1.71/1.31 | 26.13 |
| 5 | 1.82/1.67 | 27.11 |
| 6 | 1.84/1.32 | 30.80 |
| 7 | 4.55 | 51.14 |
| 8 | 7.84 | - |
| 9 | - | 169.54 |
| 10 | 3.69 | 81.55 |
| 10-OMe | 3.25 | 57.28 |
| 11 | 3.57 | 70.69 |
| 11-OH | 4.45 | - |
| 12 | 3.33 | 72.77 |
| 12-OH | 4.37 | - |
| 13 | 3.97 | 72.47 |
| 13-OH | 4.56 | - |
| 14 | 5.37 | 129.04 |
| 15 | 5.48 | 136.58 |
| 16 | 1.99 | 37.41 |
| 16-Me | 0.93 | 19.89 |
| 17 | 1.26 | 29.15 |
| 18 | 0.81 | 11.58 |

### Beispiel 14: Charakterisierung der Verbindung der Formel (IV)

Summenformel: C₁₈H₃₂N₂O₇
Molekulargewicht: 388,46
Diastereomeren-Gemisch: 75/25

| Tabelle 4: NMR-chemische Verschiebungen von Bengamide (IV), Diastereomerengemisch, c = 3.1 mg/ml in DMSO, 300 K. | | |
|---|---|---|
| Position | ¹H | ¹³C |
| 1 | - | 173.45 |
| 2 | 7.55 | - |
| 3 | 3.35/3.02 | 45.05 |
| 4 | 3.74 | 63.48 |
| 4-OH | 4.60 - | - |
| 5 | 1.81/1.75 | 34.28 |
| 6 | 1.68/1.64 | 24.25 |
| 7 | 4.32 | 51.18 |
| 8 | 7.77 | - |
| 9 | - | 169.63 |
| 10 | 3.70 | 81.59 |
| 10-OMe | 3.26 | 57.30 |
| 11 | 3.58 | 70.73 |
| 11-OH | 4.47 | - |
| 12 | 3.34 | 72.82 |
| 12-OH | 4.38 | - |
| 13 | 3.97 | 72.47 |
| 13-OH | 4.56 | - |
| 14 | 5.37 | 129.06 |
| 15 | 5.48 | 136.57 |
| 16 | 1.99 | 37.41 |
| 16-Me | 0.93 | 19.90 |
| 17 | 1.26 | 29.15 |
| 18 | 0.81 | 11.58 |

### Beispiel 15: Charakterisierung von Bengamide E (Vergleichsversuch)

Summenformel: C₁₇H₃₀N₂O₆
Molekulargewicht: 358,44

| Tabelle 5: NMR-chemische Verschiebungen von Bengamide E, c = 3 mg/ml in DMSO, 300 K. | | |
|---|---|---|
| Position | ¹H | ¹³C |
| 1 | - | 174.01 |
| 2 | 7.91 | - |
| 3 | 3.19/3.06 | 40.56 |
| 4 | 1.74/1.20 | 28.75 |
| 5 | 1.87/1.64 | 27.56 |
| 6 | 1.87/1.36 | 30.72 |
| 7 | 4.39 | 51.27 |
| 8 | 7.78 | - |
| 9 | - | 169.60 |
| 10 | 3.69 | 81.61 |
| 10-OMe | 3.25 | 57.30 |
| 11 | 3.56 | 70.74 |
| 11-OH | 4.49 | - |
| 12 | 3.33 | 72.78 |
| 12-OH | 4.38 | - |
| 13 | 3.96 | 72.38 |
| 13-OH | 4.57 | - |
| 14 | 5.38 | 127.68 |
| 15 | 5.58 | 137.85 |
| 16 | 2.24 | 30.08 |
| 17 | 0.95 | 22.27 |
| 18 | 0.95 | 22.17 |

### Beispiel 16: Charakterisierung von Bengamide F (Vergleichsversuch)

Summenformel: C₁₈H₃₂N₂O₆
Molekulargewicht: 372,47

| Tabelle 6: NMR-chemische Verschiebungen von Bengamide F, c = 3 mg/ml in DMSO, 300 K. | |
|---|---|
| Position | ¹H |
| 1 | - |
| 2 | 2.91 |
| 3 | 3.62/3.21 |
| 4 | 1.69/1.33 |
| 5 | 1.84/1.69 |
| 6 | 1.84/1.33 |
| 7 | 4.56 |
| 8 | 7.84 |
| 9 | - |
| 10 | 3.70 |
| 10-OMe | 3.25 |
| 11 | 3.57 |
| 12 | 3.33 |
| 13 | 3.97 |
| 14 | 5.38 |
| 15 | 5.59 |
| 16 | 2.25 |
| 17 | 0.95 |
| 18 | 0.95 |

### Beispiel 17: Trennung der Diastereomeren von Verbindung (II)

Die Trennung des Diastereomerengemischs der Verbindung der Formel (II) aus Beispiel 8 erfolgte an einer chiralen Säule (AD/H, Daicel, 20 x 200mm, 0,5 ml Fluss, Laufmittel: Acetonitril:Methanol 4:1 + 0,1 % NH₄Ac). Die optische Reinheit wurde auf einer analytischen AD/H-Säule (Daicel) kontrolliert (4,6 x 250mm, 30°C, Laufmittel: Acetonitril:Methanol 4:1 + 0,1% NH₄Ac, 0,75 ml Fluss, Rt Peak1: 9,9 min Rt Peak2: 10,9 min).

| Tabelle 7: NMR-chemische Verschiebungen der Diastereomere von Bengamide (II), c = 3 mg/ml in DMSO, 300 K. | | | | |
|---|---|---|---|---|
| | ¹H (A) | ¹H (B) | ¹³C (A) | ¹³C (B) |
| 1 | - | - | 173.99 | 173.99 |
| 2 | 7.91 | 7.91 | - | - |
| 3 | 3.19/3.06 | 3.19/3.06 | 40.56 | 40.56 |
| 4 | 1.74/1.20 | 1.74/1.20 | 28.75 | 28.75 |
| 5 | 1.87/1.64 | 1.87/1.64 | 27.55 | 27.55 |
| 6 | 1.87/1.36 | 1.87/1.36 | 30.72 | 30.72 |
| 7 | 4.39 | 4.39 | 51.27 | 51.27 |
| 8 | 7.78 | 7.78 | - | - |
| 9 | - | - | 169.61 | 169.61 |
| 10 | 3.69 | 3.69 | 81.60 | 81.60 |
| 10-OMe | 3.25 | 3.25 | 57.32 | 57.32 |
| 11 | 3.58 | 3.58 | 70.72 | 70.77 |
| 11-OH | 4.46 | 4.47 | - | - |
| 12 | 3.33 | 3.33 | 72.80 | 72.85 |
| 12-OH | 4.36 | 4.36 | - | - |
| 13 | 3.97 | 3.97 | 72.46 | 72.37 |
| 13-OH | 4.56 | 4.56 | - | - |
| 14 | 5.37 | 5.38 | 129.05 | 129.01 |
| 15 | 5.48 | 5.49 | 136.57 | 136.44 |
| 16 | 1.99 | 1.99 | 37.41 | 37.28 |
| 16-Me | 0.93 | 0.92 | 19.90 | 19.86 |
| 17 | 1.26 | 1.26 | 29.15 | 29.06 |
| 18 | 0.81 | 0.82 | 11.58 | 11.48 |

### Beispiel 18: Zell-Proliferationsmessungen an verschiedenen Tumorzelllinien

Zur Bestimmung der Zellproliferation wurden die Tumorzelllinien Hep-G2 (ATCC No. HB-8065) und COLO 205 (ATCC No. CCL-222) verwendet. Die Zelllinien wurden mit 1000 Zellen/well [Hep-G2] beziehungsweise 3500 Zellen/well [Colo205] in Zellkulturmedium ausgesät und 4 Stunden bei 37°C und 5% CO₂ inkubiert. Medium für Hep-G2: Dulbecco's modified Eagle's medium / Ham's F12-Mix (Gibco); NEAA (10% ; non essential amino acids, Gibco), Natrium-Pyruvat (1%, Gibco), L-Glutamin (1%, Gibco), fetales Kälberserum (5%; PAA)].

Medium für COLO 205: RPMI 1640 (Gibco), L-Glutamin (1%, Gibco), HEPES (1%, Gibco), fetales Kälberserum (10%; PAA).

Nach 4 Stunden wurden die Verbindungen (II), (III), (IV) und die Bengamide E und F, gelöst in DMSO/Zellkulturmedium, in verschiedenen Verdünnungen zugegeben und 72 Stunden bei 37°C und 5% CO₂ inkubiert. Die intrazelluläre ATP-Gehaltsbestimmung erfolgte unter Verwendung des Testreagenz CellTiterGlo (Promega).

Die Ergebnisse der Zellproliferationstests sind in Tabelle 1 beschrieben.

## Patentansprüche

1. Verbindung der Formel (I), 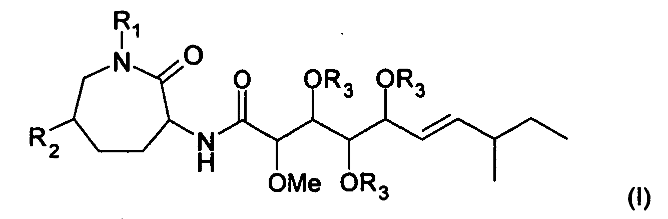 wobei
R₁ H oder (C₁-C₆)-Alkyl,
R₂ H oder OH, und
R₃ H oder -C(=O)-(C₁-C₆)-Alkyl bedeutet,
oder ein physiologisch verträgliches Salz davon.

2. Verbindung gemäß Anspruch 1, wobei R₁ H oder Methyl bedeutet.

3. Verbindung gemäß einem der Ansprüche 1 oder 2, wobei R₃ H bedeutet.

4. Verbindung gemäß einem der Ansprüche 1 bis 3, wobei R₁ H oder Methyl, R₂ H oder OH, und R₃ H bedeutet.

5. Verbindung gemäß einem der Ansprüche 1 bis 4, **gekennzeichnet durch** eine Verbindung der Formel (II)

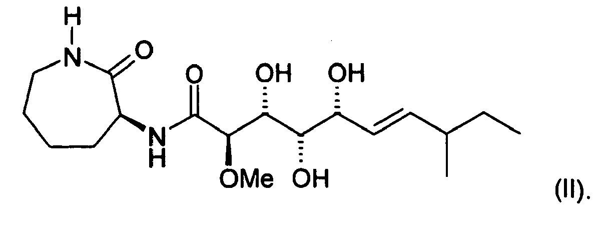

6. Verbindung gemäß einem der Ansprüche 1 bis 4, **gekennzeichnet durch** eine Verbindung der Formel (III)

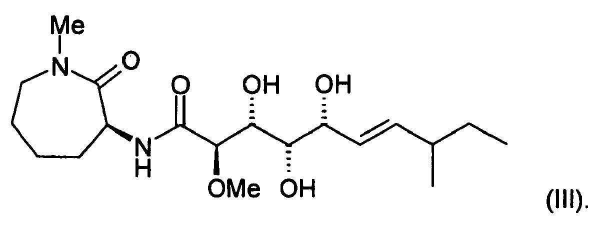

7. Verbindung gemäß einem der Ansprüche 1 bis 4, **gekennzeichnet durch** eine Verbindung der Formel (IV)

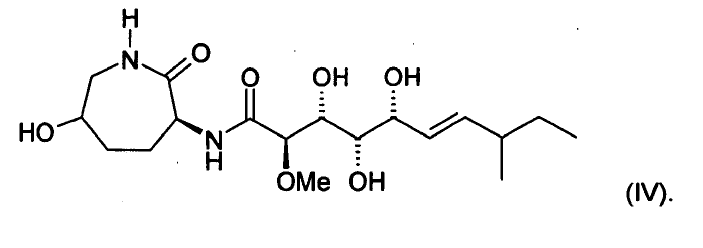

8. Verwendung einer Verbindung der Formel (I) oder eines physiologisch verträglichen Salzes davon gemäß einem der Ansprüche 1 bis 7 zur Herstellung eines Arzneimittel.

9. Verwendung einer Verbindung der Formel (I) oder eines physiologisch verträglichen Salzes davon gemäß einem der Ansprüche 1 bis 7 zur Herstellung eines Arzneimittel zur Behandlung und/oder Prophylaxe von Krebserkrankungen.

10. Arzneimittel mit einem Gehalt an mindestens einer Verbindung der Formel (I) oder eines physiologisch verträglichen Salzes davon gemäß einem der Ansprüche 1 bis 7.

## Claims

1. A compound of the formula (I), 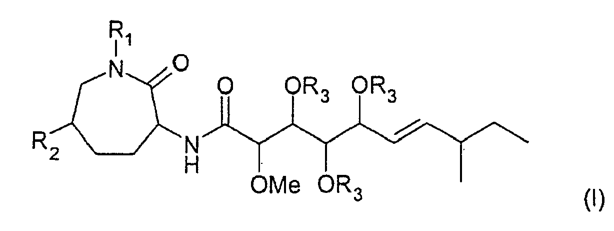 wherein
R₁ is H or (C₁-C₆)-alkyl,
R₂ is H or OH, and
R₃ is H or -C(=O)-(C₁-C₆)-alkyl,
or a physiologically tolerated salt thereof.

2. A compound as claimed in claim 1, wherein R₁ is H or methyl.

3. A compound as claimed in claim 1 or 2, wherein R₃ is H.

4. A compound as claimed in one of claims 1 to 3, wherein R₁ is H or methyl, R₂ is H or OH, and R₃ is H.

5. A compound as claimed in one of claims 1 to 4, **characterized by** a compound of the formula (II)

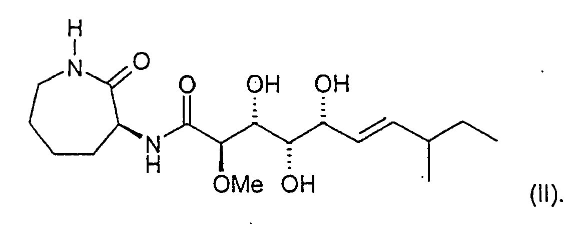

6. A compound as claimed in one of claims 1 to 4, **characterized by** a compound of the formula (III)

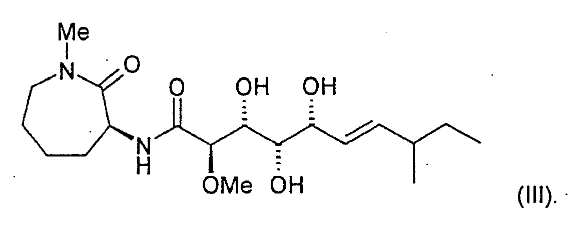

7. A compound as claimed in one of claims 1 to 4, **characterized by** a compound of the formula (IV)

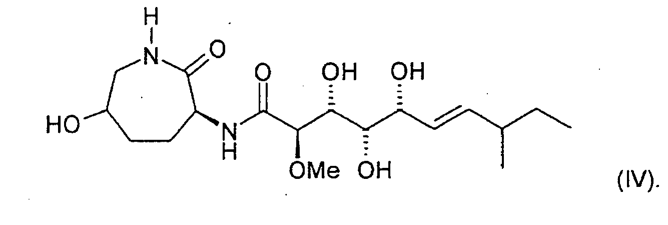

8. The use of a compound of the formula (I) or of a physiologically tolerated salt thereof as claimed in one of claims 1 to 7 in the manufacture of a pharmaceutical.

9. The use of a compound of the formula (I) or of a physiologically tolerated salt thereof as claimed in one of claims 1 to 7 in the manufacture of a pharmaceutical for the treatment and/or prophylaxis of cancer diseases.

10. A pharmaceutical having a content of at least one compound of the formula (I) or of a physiologically tolerated salt thereof as claimed in one of claims 1 to 7.

## Revendications

1. Composé de formule (I), 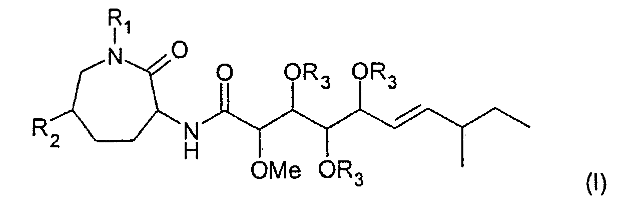 dans laquelle
R₁ représente H ou (C₁-C₆)-alkyle,
R₂ représente H ou OH, et
R₃ représente H ou -C(=O)-(C₁-C₆)-alkyle,
ou un sel physiologiquement toléré de celui-ci.

2. Composé selon la revendication 1, dans lequel R₁ représente H ou méthyle.

3. Composé selon la revendication 1 ou 2, dans lequel R₃ représente H.

4. Composé selon l'une quelconque des revendications 1 à 3, dans lequel R₁ représente H ou méthyle, R₂ représente H ou OH et R₃ représente H.

5. Composé selon l'une quelconque des revendications 1 à 4, qui est **caractérisé par** un composé de formule (II)

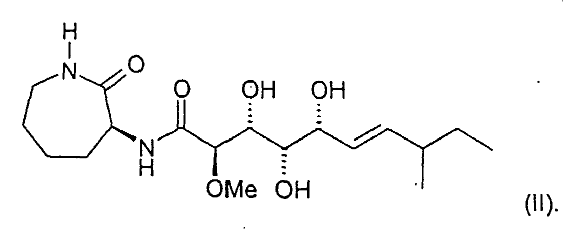

6. Composé selon l'une quelconque des revendications 1 à 4, qui est **caractérisé par** un composé de formule (III)

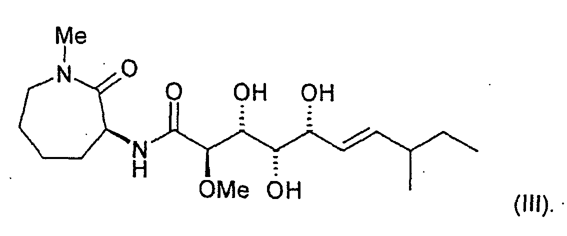

7. Composé selon l'une quelconque des revendications 1 à 4, qui est **caractérisé par** un composé de formule (IV)

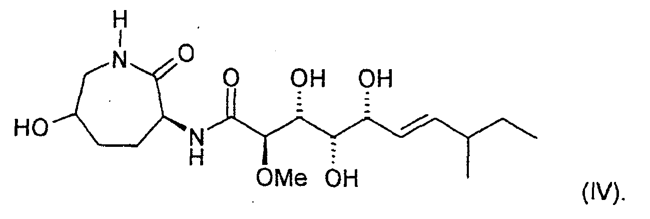

8. Utilisation d'un composé de formule (I) ou d'un sel physiologiquement toléré de celui-ci selon l'une quelconque des revendications 1 à 7 pour préparer un produit pharmaceutique.

9. Utilisation d'un composé de formule (I) ou d'un sel physiologiquement toléré de celui-ci selon l'une quelconque des revendications 1 à 7 pour préparer un produit pharmaceutique destiné au traitement et/ou à la prophylaxie de maladies cancéreuses.

10. Produit pharmaceutique présentant une teneur en au moins un composé de formule (I) ou en un sel physiologiquement toléré de celui-ci selon l'une quelconque des revendications 1 à 7.
